# EUROPEAN PATENT APPLICATION

(11) **EP 3 981 389 A1**
(43) Date of publication of application: **13.04.2022**
(21) Application number: 19931527.6
(22) Date of filing: 25.06.2019
(51) Int. Cl.: A61K 9/08, A61K 9/00, A61K 47/08, A61K 47/14, A61K 31/454

(54) **TOPICALLY ADMINISTERED PHARMACEUTICAL COMPOSITION IN SOLUTION FORM CONTAINING EFINACONAZOLE**

(30) Priority: 07.06.2019 KR 20190067191
(71) Applicant: Biobelief Co., Ltd, Seoul 08225 (KR)
(72) Inventor: KIM, Beom-Joon, Seoul 06043 (KR); KOO, Kyo-Tan, Seongnam-si Gyeonggi-do 13586 (KR)
(74) Representative: Turner, Craig Robert
(86) International application number: PCT/KR2019/007616
(87) International publication number: WO 2020/246654

(57) **Abstract**

The present invention provides an efinaconazole-containing pharmaceutical composition for topical administration in the form of solution comprising diethylene glycol monoethyl ether; or a combination of diethylene glycol monoethyl ether and isopropyl myristate as a non-volatile solvent. The pharmaceutical composition of the present invention can remarkably increase the skin and nail penetration rate and permeability (flux) of efinaconazole, and also has excellent stability.

## Description

### TECHNICAL FIELD

The present invention relates to a pharmaceutical composition for topical administration in the form of solution comprising efinaconazole. More specifically, the present invention relates to a pharmaceutical composition for topical administration in the form of solution comprising diethylene glycol monoethyl ether; or a combination of diethylene glycol monoethyl ether and isopropyl myristate as a non-volatile solvent.

### BACKGROUND ART

Efinaconazole is a triazole antifungal agent having the following chemical structure of Formula 1 and the chemical name thereof is (2R,3R)-2-(2,4-difluorophenyl)-3-(4-methylenepiperidin-1-yl)-1-(1H-1,2,4-triazol-1-yl)but an-2-ol.

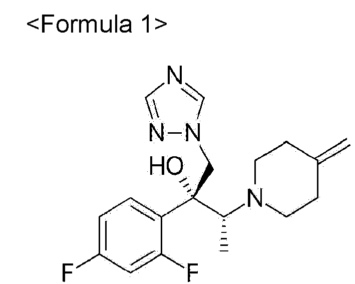

Efinaconazole has an inhibitory activity against lanosterol 14α-demethylase in the ergosterol biosynthetic pathway; and is marketed as a 10% topical solution for the treatment of onychomycosis (Trade name: JUBLIA^{™}, Kaken Pharmaceutical Co., Ltd.). The topical solution includes, along with efinaconazole, ethanol as a volatile solvent; cyclomethicone as a wetting agent; and diisopropyl adipate and C₁₂-C₁₅ alkyl lactate as non-volatile solvents (e.g., US Patent Nos. 7,214,506, 8,039,494, 8,486,978, 9,302,009, 9,566,272, 9,861,698, 9,877,955, and the like).

Efinaconazole-containing solutions have a stability problem, i.e., the problem to discolor within short storage periods, resulting in composition colors ranging from yellow to deep red or brown. In order to solve the problem, US Patent No. 9,662,394 and International Laid-open Publication No. WO2015/051183 have disclosed a liquid or semisolid composition comprising the specific combinations of a chelating agent, an antioxidant, and an acid, that is, a liquid or semisolid composition comprising ethylenediaminetetraacetic acid (EDTA) or a salt thereof, butylated hydroxytoluene (BHT), and citric acid.

Meanwhile, a semisolid formulation such as gels, ointments, and creams has an advantage of reducing the amounts of efinaconazole in the formulations, since the duration residing on the skin and nail after the application thereof is relatively longer in comparison with a formulation in the form of solution. However, since the semisolid formulation such as gels, ointments, and creams includes a thickening agent (e.g., acrylates/alkyl acrylate crosspolymer), it has sticky or gluey properties. And also, in order to be absorbed through the skin or nail, a semisolid formulation takes longer time than an external liquid formulation. Therefore, a semisolid formulation such as gels, ointments, and creams may cause inconvenience in daily life of the subjects after the application thereof, which results in poor drug compliance.

Since a formulation in the form of solution contains a volatile solvent such as ethanol as a vehicle, it does not exhibit sticky or gluey properties after the application thereof. And also, a formulation in the form of solution is dried in a short time and thus has an advantage that there is no inconvenience in daily life. However, conventional formulations in the form of solution, including JUBLIA^{™} (Kaken Pharmaceutical Co., Ltd.), are still unsatisfactory in the skin or nail penetration rate and permeability (flux) of the drug, relatively to the volatilization rates of solvent after the application thereof. In addition, conventional efinaconazole-containing external formulations, including JUBLIA^{™} (Kaken Pharmaceutical Co., Ltd.), require using the specific combinations of a chelating agent, an antioxidant and an acid, in order to ensure the stability of the drug therein. Therefore, conventional efinaconazole-containing external formulations are limited in the design of various forms of external formulations.

### DISCLOSURE

### Technical Problem

The present inventors carried out various researches in order to develop an efinaconazole-containing formulation for topical administration in the form of solution, which can improve the skin and nail penetration rate and permeability thereof. As the results thereof, the present inventors have found that the skin or nail penetration rate and permeability (flux) of efinaconazole can be remarkably increased when the formulating are carried out by using diethylene glycol monoethyl ether; or a combination of diethylene glycol monoethyl ether and isopropyl myristate, instead of diisopropyl adipate and C₁₂-C₁₅ alkyl lactate, as a non-volatile solvent. And also, it has been found that a formulation in the form of solution comprising diethylene glycol monoethyl ether; or a combination of diethylene glycol monoethyl ether and isopropyl myristate as a non-volatile solvent has stability equivalent to the known formulation comprising EDTA/BHT/citric acid, even when various combinations of a chelating agent, an antioxidant, and an acid are used.

Therefore, it is an object of the present invention to provide a pharmaceutical composition for topical administration in the form of solution comprising diethylene glycol monoethyl ether; or a combination of diethylene glycol monoethyl ether and isopropyl myristate as a non-volatile solvent.

### Technical Solution

In accordance with an aspect of the present invention, there is provided a pharmaceutical composition for topical administration in the form of solution comprising efinaconazole, ethanol, cyclomethicone, and a non-volatile solvent, characterized in that the non-volatile solvent is diethylene glycol monoethyl ether; or a combination of diethylene glycol monoethyl ether and isopropyl myristate.

In the pharmaceutical composition of the present invention, the non-volatile solvent may be diethylene glycol monoethyl ether and the diethylene glycol monoethyl ether may be present in an amount ranging from 8 to 20 % by weight, based on the total weight of the composition.

In the pharmaceutical composition of the present invention, the non-volatile solvent may be a combination of diethylene glycol monoethyl ether and isopropyl myristate, the diethylene glycol monoethyl ether may be present in an amount ranging from 8 to 20 % by weight, based on the total weight of the composition, and the isopropyl myristate may be present in an amount ranging from 5 to 25 % by weight, based on the total weight of the composition. Preferably, the non-volatile solvent may be a combination of diethylene glycol monoethyl ether and isopropyl myristate, the diethylene glycol monoethyl ether may be present in an amount ranging from 10 to 15 % by weight, based on the total weight of the composition, and the isopropyl myristate may be present in an amount ranging from 6 to 20 % by weight, based on the total weight of the composition.

The pharmaceutical composition of the present invention may further comprises one or more excipients selected from the group consisting of a chelating agent, an antioxidant, and an acid. The chelating agent may be selected from the group consisting of ethylenediaminetetraacetic acid or a sodium salt thereof, tetrahydroxypropyl ethylenediamine, and diethylenetriaminepentaacetic acid, preferably, tetrahydroxypropyl ethylenediamine or diethylenetriaminepentaacetic acid. The antioxidant may be butylated hydroxyanisole or butylated hydroxytoluene. And also, the acid may be sorbic acid or citric acid.

### ADVANTAGEOUS EFFECTS

It has been found by the present invention that the skin and nail penetration rate and permeability (flux) of efinaconazole can be remarkably increased when the formulating are carried out by using diethylene glycol monoethyl ether; or a combination of diethylene glycol monoethyl ether and isopropyl myristate as a non-volatile solvent. Therefore, the pharmaceutical composition of the present invention can be usefully applied as a formulation for topical administration having high drug compliance and high skin or nail penetration rate and permeability (flux). In addition, the pharmaceutical composition of the present invention has excellent stability, which makes it possible to use various combinations of a chelating agent, an antioxidant, and an acid.

### DESCRIPTION OF DRAWINGS

FIGs. 1 to 3 show the results obtained from evaluating the skin and nail penetration rates and fluxes of the pharmaceutical composition of the present invention.
FIG. 1 shows the results obtained from calculating the nail fluxes based on the changes of the nail-permeated concentrations at Day 1 of the application of the pharmaceutical compositions of the present invention.
FIG. 2 shows the results obtained from measuring the changes of the nail-permeated concentrations during the consecutive application of the pharmaceutical compositions of the present invention for 1 week.
FIG. 3 shows the results obtained from calculating the nail fluxes based on the results of FIG. 2.

### BEST MODE

The present invention provides a pharmaceutical composition for topical administration in the form of solution comprising efinaconazole, ethanol, cyclomethicone, and a non-volatile solvent, characterized in that the non-volatile solvent is diethylene glycol monoethyl ether; or a combination of diethylene glycol monoethyl ether and isopropyl myristate.

The pharmaceutical composition of the present invention comprises efinaconazole as an active ingredient. Efinaconazole may be used in a therapeutically effective amounts, for example in the range of 8 to 12 % by weight, preferably about 10% by weight, based on the total weight of the composition, but is not limited thereto.

The pharmaceutical composition of the present invention comprises diethylene glycol monoethyl ether; or a combination of diethylene glycol monoethyl ether and isopropyl myristate as a non-volatile solvent, thereby being able to remarkably increase the skin and nail penetration rate of efinaconazole. As said diethylene glycol monoethyl ether, commercially available Transcutol^{™} P (Gattefosse) may be also used. In the pharmaceutical composition of the present invention, the non-volatile solvent may be preferably diethylene glycol monoethyl ether and the diethylene glycol monoethyl ether may be present in an amount ranging from 8 to 20 % by weight, based on the total weight of the composition. In addition, in the pharmaceutical composition of the present invention, the non-volatile solvent may be preferably a combination of diethylene glycol monoethyl ether and isopropyl myristate, the diethylene glycol monoethyl ether may be present in an amount ranging from 8 to 20 % by weight, based on the total weight of the composition, and the isopropyl myristate may be present in an amount ranging from 5 to 25 % by weight, based on the total weight of the composition. More preferably, the non-volatile solvent may be a combination of diethylene glycol monoethyl ether and isopropyl myristate, the diethylene glycol monoethyl ether may be present in an amount ranging from 10 to 15 % by weight, based on the total weight of the composition, and the isopropyl myristate may be present in an amount ranging from 6 to 20 % by weight, based on the total weight of the composition.

In an embodiment, there is provided a pharmaceutical composition for topical administration in the form of solution, comprising 8 to 12 % by weight of efinaconazole, 50 to 65 % by weight of ethanol, 8 to 20 % by weight of cyclomethicone, and 8 to 20 % by weight of diethylene glycol monoethyl ether. In another embodiment, there is provided a pharmaceutical composition for topical administration in the form of solution, comprising 8 to 12 % by weight of efinaconazole, 40 to 65 % by weight of ethanol, 8 to 20 % by weight of cyclomethicone, 8 to 20 % by weight of diethylene glycol monoethyl ether, and 5 to 25 % by weight of isopropyl myristate. In still another embodiment, there is provided a pharmaceutical composition for topical administration in the form of solution, comprising 8 to 12 % by weight of efinaconazole, 40 to 65 % by weight of ethanol, 8 to 20 % by weight of cyclomethicone, 10 to 15 % by weight of diethylene glycol monoethyl ether, and 6 to 20 % by weight of isopropyl myristate.

The pharmaceutical composition of the present invention has excellent stability and therefore has equivalent stability to the known formulation comprising EDTA/BHT/citric acid, even when various combinations of a chelating agent, an antioxidant, and an acid are used. Therefore, the pharmaceutical composition of the present invention may further comprise one or more excipients selected from the group consisting of a chelating agent, an antioxidant, and an acid. And also, if necessary, the pharmaceutical composition of the present invention may further comprise a small amount (e.g., about 1.0 % by weight or less) of water.

The chelating agent may be selected from the group consisting of ethylenediaminetetraacetic acid (EDTA) or a sodium salt thereof, tetrahydroxypropyl ethylenediamine, and diethylenetriaminepentaacetic acid. Preferably, the chelating agent may be tetrahydroxypropyl ethylenediamine or diethylenetriaminepentaacetic acid. The antioxidant may be butylated hydroxyanisole (BHA) or butylated hydroxytoluene (BHT). In addition, the acid may be sorbic acid or citric acid.

In an embodiment, there is provided a pharmaceutical composition for topical administration in the form of solution, comprising 8 to 12 % by weight of efinaconazole, 50 to 65 % by weight of ethanol, 8 to 20 % by weight of cyclomethicone, 8 to 20 % by weight of diethylene glycol monoethyl ether, 0.00025 to 0.05 % by weight of a chelating agent, 0.1 to 0.2 % by weight of an antioxidant, 0.1 to 0.2 % by weight of an acid, and 0 to 1.0 % by weight of water. In another embodiment, there is provided a pharmaceutical composition for topical administration in the form of solution, comprising 8 to 12 % by weight of efinaconazole, 40 to 65 % by weight of ethanol, 8 to 20 % by weight of cyclomethicone, 8 to 20 % by weight of diethylene glycol monoethyl ether, 5 to 25 % by weight of isopropyl myristate, 0.00025 to 0.05 % by weight of a chelating agent, 0.1 to 0.2 % by weight of an antioxidant, 0.1 to 0.2 % by weight of an acid, and 0 to 1.0 % by weight of water. In still another embodiment, there is provided a pharmaceutical composition for topical administration in the form of solution, comprising 8 to 12 % by weight of efinaconazole, 40 to 65 % by weight of ethanol, 8 to 20 % by weight of cyclomethicone, 10 to 15 % by weight of diethylene glycol monoethyl ether, 6 to 20 % by weight of isopropyl myristate, 0.00025 to 0.05 % by weight of a chelating agent, 0.1 to 0.2 % by weight of an antioxidant, 0.1 to 0.2 % by weight of an acid, and 0 to 1.0 % by weight of water.

In a particularly preferred embodiment of the invention, there is provided a pharmaceutical composition for topical administration in the form of solution, consisting of 10 % by weight of efinaconazole, 52.75 % by weight of ethanol, 15 % by weight of cyclomethicone, 12 % by weight of diethylene glycol monoethyl ether, 10 % by weight of isopropyl myristate, 0.05 % by weight of tetrahydroxypropyl ethylenediamine, 0.1 % by weight of butylated hydroxytoluene, and 0.1 % by weight of citric acid. In another particularly preferred embodiment of the invention, there is provided a pharmaceutical composition for topical administration in the form of solution, consisting of 10 % by weight of efinaconazole, 52.29975 % by weight of ethanol, 15 % by weight of cyclomethicone, 12 % by weight of diethylene glycol monoethyl ether, 10 % by weight of isopropyl myristate, 0.00025% by weight of diethylenetriaminepentaacetic acid, 0.1 % by weight of butylated hydroxytoluene, 0.1 % by weight of citric acid, and 0.5 % by weight of water.

The pharmaceutical composition of the present invention may be prepared by mixing according to a conventional method using the above-mentioned components. If necessary, the pharmaceutical composition of the present invention may be prepared by preparing a stock solution containing a chelating agent and a stock solution containing efinaconazole, respectively, followed by mixing with the other components to form a solution.

The present invention will be described in further detail with reference to the following examples and experimental examples. These examples and experimental examples are for illustrative purposes only and are not intended to limit the scope of the present invention.

The high performance liquid chromatography (HPLC) analyses in the following Examples were carried out under the following conditions.

### < Condition for HPLC analyses >

- Column: Luna C18 (150 ×4.6 mm, 5 µm)
- Column temperature: 30□
- Mobile phase: acetonitrile/water (64/36, v/v)
- Flow rate: 1.0 mL/min
- Detection: 205 nm
- Injection volume: 20 µL

And also, the LC/MS analyses were carried out as follows.

### <1> Preparation of the solutions for a calibration curve and the sample solutions

A solution of efinaconazole in ethanol (200 µL) was diluted with phosphate buffered saline (PBS) (200 µL, pH 7.4) containing 4 %(w/v) bovine serum albumin and 0.01 %(w/v) sodium azide, so as to prepare the solutions for a calibration curve having the concentrations of efinaconazole: 0, 50, 100, 250, 500, 1,000, 2,500, and 5,000 ng/mL. A solution of quercetin (QCN, 1 µg/mL, internal standard) in methanol (200 µL) was added to each solution for the calibration curve of efinaconazole (200 µL) and each sample solution (200 µL), followed by mixing the resulting solutions. Methanol (1 mL) and ethanol (1 mL) were added to each resulting solution for a calibration curve and each sample solution, followed by mixing the resulting solutions. The resulting solutions were centrifuged at 4,000 rpm for 10 minutes. Each supernatant thereof was transferred into a glass vial and then dried with a rotary evaporator to remove the solvent. To each resulting glass vial, were added 100 µL of ethanol and 100 µL of the mobile phase (acetonitrile : de-ionized water, 64:36, v/v), so as to re-dissolve the components. The resulting solutions were centrifuged at 10,000 rpm for 10 minutes. Each supernatant thereof was taken and then subject to LC/MS analyses under the following conditions to measure the concentrations of efinaconazole therein.

### <2> Condition of LC analyses

- Column: Luna C18 (150 ×4.6 mm, 5 µm)
- Column temperature: 30°C
- Mobile phase: acetonitrile/water (64/36, v/v)
- Flow rate: 1.0 mL/min
- Detection: 205 nm
- Injection volume: 20 µL

### <3> Condition of Mass analyses

- Efinaconazole: positive ionization mode
- Quercetin: negative ionization mode
- Source: API-ES (atmospheric pressure ionization-electron spray)
- Capillary voltage: 3,000 V
- Drying gas flow rate: 12.0 L/min
- Drying gas temperature: 304□
- The fragment ions at the capillary voltage were detected and quantified at [M+H]⁺=349.3 and [M+H]⁺=301.0 for efinaconazole and quercetin, respectively.

### Examples 1 to 7. Preparation of solutions and evaluation of skin permeabilities thereof

### (1) Preparation of solutions

The efinaconazole-containing solutions were prepared according to the components and amounts shown in Table 1. The amounts of Table 1 represent % by weight of each component in the solution. Ethylenediaminetetraacetic acid disodium salt (EDTA disodium salt) was dissolved in sterile water in the concentration of 0.025 mg/mL to prepare a stock solution. And also, efinaconazole (0.2 g) was dissolved in ethanol (0.076 g) to prepare an efinaconazole-containing stock solution. Cyclomethicone, Transcutol^{™} P (Gattefosse), isopropyl myristate, butylated hydroxyanisole (BHA), sorbic acid, and EDTA disodium salt (added in the form of a stock solution) were one by one added to the efinaconazole-containing solution in ethanol, followed by mixing each resulting solution, to prepare the efinaconazole-containing solutions.

**<Table 1>**

| | Example (% by weight) | | | | | | |
|---|---|---|---|---|---|---|---|
| | 1 | 2 | 3 | 4 | 5 | 6 | 7 |
| Efinaconazole | 10 | 10 | 10 | 10 | 10 | 10 | 10 |
| Ethanol | 56.79975 | 51.79975 | 55.79975 | 50.79975 | 59.79975 | 50.79975 | 45.79975 |
| Cyclomethicone | 10 | 15 | 13 | 13 | 13 | 13 | 13 |
| Transcutol^{™} P | 12 | 12 | 10 | 15 | 10 | 10 | 10 |
| Isopropyl myristate | 10 | 10 | 10 | 10 | 6 | 15 | 20 |
| EDTA disodium salt | 0.00025 | 0.00025 | 0.00025 | 0.00025 | 0.00025 | 0.00025 | 0.00025 |
| BHA | 0.1 | 0.1 | 0.1 | 0.1 | 0.1 | 0.1 | 0.1 |
| Sterile water | 1 | 1 | 1 | 1 | 1 | 1 | 1 |
| Sorbic acid | 0.1 | 0.1 | 0.1 | 0.1 | 0.1 | 0.1 | 0.1 |
| Total (%) | 100 | 100 | 100 | 100 | 100 | 100 | 100 |

### (2) Evaluation of skin permeabilities using an artificial skin

The skin permeabilities of the solutions prepared in Examples 1 to 7 were evaluated, in a Franz diffusion cell, using an artificial skin. As a control formulation, JUBLIA^{™} (efinaconazole 10% containing-topical solution, Kalen Pharmaceutical Co., Ltd.) was used. As an artificial skin, Strat-M^{™} Membrane (transdermal diffusion test membrane, Millipore) was used. The artificial skin was sufficiently hydrated for more than 1 hour and then 200 µL of each solution was applied to the respective donor compartments. The skin permeability tests using the Franz diffusion cell were carried out four times (n = 4) under the following conditions.
- Diffusion area: 0.785 cm²
- Sample loading: 200 µL of each solution prepared in Examples 1 to 7
- Receiver compartment: 5 mL of PBS (pH 7.4) containing 1.5 %(w/v) Brij^{™} O20 (Sigma-Aldrich) and 0.1 %(w/v) sodium azide
- Rotation speed of the receiver compartment: 600 rpm
- Temperature: 32□
- Sampling: the sample (200 µL) from each receiver compartment was taken at 12 hours and 24 hours; and then the receiver compartment was refilled with 200 µL of PBS containing 1.5 %(w/v) Brij^{™} O20 (Sigma-Aldrich) and 0.1 %(w/v) sodium azide.

The obtained samples were analyzed with HPLC. The results thereof are shown in the following table 2.

**<Table 2>**

| Formulation | 12 hours | 24 hours |
|---|---|---|
| | Permeated concentration (µg/mL) | Permeated concentration (µg/mL) |
| Control formulation | 146 ± 21.8 | 326 ± 29.0 |
| Example 1 | 475 ± 42.8 | 548 ± 12.2 |
| Example 2 | 825 ± 22.1 | 841 ± 13.4 |
| Example 3 | 770 ± 199 | 832 ± 177 |
| Example 4 | 689 ± 141 | 783 ± 80.5 |
| Example 5 | 783 ± 20.5 | 805 ± 34.8 |
| Example 6 | 762 ± 100 | 891 ± 9.48 |
| Example 7 | 608 ± 119 | 717 ± 74.6 |

As can be seen from the results of Table 2, the skin permeabilities of the solutions obtained according to the present invention were remarkably increased as compared with the control formulation. Especially, the solutions of Examples 2 and 5 showed a 5-times more increase in the skin permeability for 12 hours, compared to the control formulation.

### (3) Evaluation of skin permeability using a human skin

The skin permeability of the solution prepared in Example 2 was evaluated, in a Franz diffusion cell, using a human skin. As a control formulation, JUBLIA^{™} (efinaconazole 10% containing-topical solution, Kalen Pharmaceutical Co., Ltd.) was used. As a human skin, HuSkin^{™} (HansBiomed Corp.) was used. The human skin was sufficiently hydrated for more than 1 hour and then 200 µL of the solution was applied to the respective donor compartments. The skin permeability tests were carried out four times (n = 4) under the same conditions as in the above skin permeability tests using an artificial skin.

The obtained samples were analyzed with HPLC. The results thereof are shown in the following table 3.

**<Table 3>**

| Formulation | Permeated concentration for 24 hours (µg/mL) |
|---|---|
| Control formulation | 16.91 ± 1.03 |
| Example 2 | 22.49 ± 2.14 |

As can be seen from the results of Table 3, the solution obtained according to the present invention showed a 2-times more increase in the skin permeability for 24 hours, compared to the control formulation.

### (4) Stability evaluation

The solutions prepared in Examples 1 to 7 were respectively stored at 65□ for 4 weeks. The absorbance values of the initial solution (0 week) and the solution stored for 4 weeks were measured at 400 nm, 500 nm, and 600 nm. The stability evaluations were performed using the cutoff values of 0.4 AU (absorbance units) or less at 400 nm, 0.1 AU or less at 500 nm, and 0.1 AU or less at 600 nm. The results thereof are shown in the following tables 4 to 6.

**<Table 4>**

| Absorbance at 400 nm | | |
|---|---|---|
| | 0 week | 4 weeks |
| Example 1 | 0.0532 ± 0.0002 | 0.060 ± 0.0020 |
| Example 2 | 0.0527 ± 0.0003 | 0.057 ± 0.0011 |
| Example 3 | 0.0536 ± 0.0004 | 0.061 ± 0.0047 |
| Example 4 | 0.0534 ± 0.0004 | 0.059 ± 0.0008 |
| Example 5 | 0.0530 ± 0.0000 | 0.058 ± 0.0023 |
| Example 6 | 0.0533 ± 0.0003 | 0.061 ± 0.0006 |
| Example 7 | 0.0535 ± 0.0001 | 0.065 ± 0.0006 |

**<Table 5>**

| Absorbance at 500 nm | | |
|---|---|---|
| | 0 week | 4 weeks |
| Example 1 | 0.0424 ± 0.0002 | 0.047 ± 0.0022 |
| Example 2 | 0.0423 ± 0.0005 | 0.045 ± 0.0015 |
| Example 3 | 0.0437 ± 0.0005 | 0.048 ± 0.0048 |
| Example 4 | 0.0419 ± 0.0004 | 0.046 ± 0.0010 |
| Example 5 | 0.0423 ± 0.0010 | 0.047 ± 0.0018 |
| Example 6 | 0.0421 ± 0.0005 | 0.048 ± 0.0010 |
| Example 7 | 0.0421 ± 0.0008 | 0.051 ± 0.0004 |

**<Table 6>**

| Absorbance at 600 nm | | |
|---|---|---|
| | 0 week | 4 weeks |
| Example 1 | 0.0407 ± 0.0003 | 0.043 ± 0.0017 |
| Example 2 | 0.0403 ± 0.0005 | 0.042 ± 0.0016 |
| Example 3 | 0.0409 ± 0.0001 | 0.046 ± 0.0046 |
| Example 4 | 0.0403 ± 0.0003 | 0.042 ± 0.0008 |
| Example 5 | 0.0399 ± 0.0004 | 0.044 ± 0.0022 |
| Example 6 | 0.0412 ± 0.0005 | 0.044 ± 0.0013 |
| Example 7 | 0.0410 ± 0.0009 | 0.045 ± 0.0006 |

From the results of Tables 4 to 6, it can be seen that the solutions obtained according to the present invention have excellent stability without changing the content of the active ingredient at the storage condition of 65°C. Especially, even when butylated hydroxyanisole was used instead of butylated hydroxytoluene and sorbic acid was used instead of citric acid, the solutions obtained according to the present invention have excellent stability without changing the content of the active ingredient.

### Examples 8 to 21. Preparation of solutions and stability evaluation thereof

### (1) Preparation of solutions

The efinaconazole-containing solutions were prepared according to the components and amounts shown in Tables 7 and 8, in the same procedures as in Examples 1 to 7. The amounts of Tables 7 and 8 represent % by weight of each component in the solution. In Tables 7 and 8, EDTA disodium represents ethylenediaminetetraacetic acid disodium salt; TED represents tetrahydroxypropyl ethylenediamine; DTPA represents diethylenetriaminepentaacetic acid; BHA represents butylated hydroxyanisole; and BHT represents butylated hydroxytoluene.

**<Table 7>**

| | Example (% by weight) | | | | | | | |
|---|---|---|---|---|---|---|---|---|
| | 8 | 9 | 10 | 11 | 12 | 13 | 14 | 15 |
| Efinaconazole | 10 | 10 | 10 | 10 | 10 | 10 | 10 | 10 |
| Ethanol | 51.6995 | 59.7998 | 52.75 | 52.75 | 51.79975 | 60.75 | 60.75 | 59.79975 |
| Cyclomethicone | 15 | 13 | 15 | 15 | 15 | 13 | 13 | 13 |
| Transcutol^{™} P | 12 | 10 | 12 | 12 | 12 | 10 | 10 | 10 |
| Isopropyl myristate | 10 | 10 | 10 | 10 | 10 | 6 | 6 | 6 |
| EDTA disodium | 0.0005 | 0.0005 | 0 | 0 | 0.00025 | 0 | 0 | 0.00025 |
| TED | 0 | 0 | 0.05 | 0.05 | 0 | 0.05 | 0.05 | 0 |
| DTPA | 0 | 0 | 0 | 0 | 0 | 0 | 0 | 0 |
| BHA | 0.2 | 0.2 | 0 | 0.1 | 0.1 | 0 | 0.1 | 0.1 |
| BHT | 0 | 0 | 0.1 | 0 | 0 | 0.1 | 0 | 0 |
| Sterile water | 1 | 1 | 0 | 0 | 1 | 0 | 0 | 1 |
| Sorbic acid | 0.1 | 0.1 | 0 | 0 | 0 | 0 | 0 | 0 |
| Citric acid | 0 | 0 | 0.1 | 0.1 | 0.1 | 0.1 | 0.1 | 0.1 |
| Total (%) | 100 | 100 | 100 | 100 | 100 | 100 | 100 | 100 |

**<Table 8>**

| | Example (% by weight) | | | | | |
|---|---|---|---|---|---|---|
| | 16 | 17 | 18 | 19 | 20 | 21 |
| Efinaconazole | 10 | 10 | 10 | 10 | 10 | 10 |
| Ethanol | 52.29975 | 52.29975 | 52.29975 | 52.29975 | 62.29975 | 60.29975 |
| Cyclomethicone | 15 | 15 | 15 | 15 | 15 | 13 |
| Transcutol^{™} P | 12 | 12 | 12 | 12 | 12 | 10 |
| Isopropyl myristate | 10 | 10 | 10 | 10 | 0 | 6 |
| EDTA disodium | 0 | 0 | 0 | 0 | 0 | 0 |
| TED | 0 | 0 | 0 | 0 | 0 | 0 |
| DTPA | 0.00025 | 0.00025 | 0.00025 | 0.00025 | 0.00025 | 0.00025 |
| BHA | 0 | 0 | 0.1 | 0.1 | 0 | 0 |
| BHT | 0.1 | 0.1 | 0 | 0 | 0.1 | 0.1 |
| Sterile water | 0.5 | 0.5 | 0.5 | 0.5 | 0.5 | 0.5 |
| Sorbic acid | 0 | 0.1 | 0 | 0.1 | 0 | 0.1 |
| Citric acid | 0.1 | 0 | 0.1 | 0 | 0.1 | 0 |
| Total (%) | 100 | 100 | 100 | 100 | 100 | 100 |

### (2) Stability evaluation

The solutions prepared in Examples 8 to 21 were respectively stored at 65°C for 4 weeks. The absorbance values were measured in the same procedures as in (4) of Examples 1 to 7. The results thereof are shown in the following tables 9 to 11.

**<Table 9>**

| Absorbance at 400 nm | | |
|---|---|---|
| | 0 week | 4 weeks |
| Example 8 | 0.0537 ± 0.0002 | 0.0878 ± 0.0022 |
| Example 9 | 0.0545 ± 0.0017 | 0.0996 ± 0.0090 |
| Example 10 | 0.0526 ± 0.0005 | 0.0579 ± 0.0008 |
| Example 11 | 0.0571 ± 0.0009 | 0.0750 ± 0.0017 |
| Example 12 | 0.0536 ± 0.0005 | 0.0751 ± 0.0025 |
| Example 13 | 0.0528 ± 0.0014 | 0.0565 ± 0.0011 |
| Example 14 | 0.0539 ± 0.0021 | 0.0581 ± 0.0007 |
| Example 15 | 0.0534 ± 0.0005 | 0.0884 ± 0.0018 |
| Example 16 | 0.0538 ± 0.0013 | 0.0541 ± 0.0004 |
| Example 17 | 0.0534 ± 0.0004 | 0.0587 ± 0.0018 |
| Example 18 | 0.0532 ± 0.0021 | 0.0592 ± 0.0075 |
| Example 19 | 0.0540 ± 0.0012 | 0.0483 ± 0.0007 |
| Example 20 | 0.0545 ± 0.0026 | 0.0527 ± 0.0019 |
| Example 21 | 0.0538 ± 0.0009 | 0.8537 ± 0.1217 |

**<Table 10>**

| Absorbance at 500 nm | | |
|---|---|---|
| | 0 week | 4 weeks |
| Example 8 | 0.0433 ± 0.0003 | 0.0653 ± 0.0018 |
| Example 9 | 0.0447 ± 0.0019 | 0.0749 ± 0.0076 |
| Example 10 | 0.0431 ± 0.0005 | 0.0468 ± 0.0016 |
| Example 11 | 0.0447 ± 0.0017 | 0.0523 ± 0.0012 |
| Example 12 | 0.0451 ± 0.0012 | 0.0529 ± 0.0012 |
| Example 13 | 0.0419 ± 0.0002 | 0.0439 ± 0.0013 |
| Example 14 | 0.0437 ± 0.0023 | 0.0448 ± 0.0009 |
| Example 15 | 0.0433 ± 0.0001 | 0.0597 ± 0.0005 |
| Example 16 | 0.0436 ± 0.0022 | 0.0430 ± 0.0002 |
| Example 17 | 0.0427 ± 0.0002 | 0.0446 ± 0.0007 |
| Example 18 | 0.0436 ± 0.0027 | 0.0452 ± 0.0017 |
| Example 19 | 0.0427 ± 0.0010 | 0.0429 ± 0.0009 |
| Example 20 | 0.0440 ± 0.0037 | 0.0467 ± 0.0019 |
| Example 21 | 0.0435 ± 0.0013 | 0.0508 ± 0.0024 |

**<Table 11>**

| Absorbance at 600 nm | | |
|---|---|---|
| | 0 week | 4 weeks |
| Example 8 | 0.0409 ± 0.0008 | 0.0432 ± 0.0005 |
| Example 9 | 0.0426 ± 0.0021 | 0.0563 ± 0.0080 |
| Example 10 | 0.0422 ± 0.0007 | 0.0421 ± 0.0010 |
| Example 11 | 0.0422 ± 0.0022 | 0.0423 ± 0.0008 |
| Example 12 | 0.0425 ± 0.0016 | 0.0444 ± 0.0015 |
| Example 13 | 0.0403 ± 0.0006 | 0.0407 ± 0.0009 |
| Example 14 | 0.0412 ± 0.0003 | 0.0419 ± 0.0007 |
| Example 15 | 0.0423 ± 0.0015 | 0.0430 ± 0.0003 |
| Example 16 | 0.0414 ± 0.0024 | 0.0407 ± 0.0003 |
| Example 17 | 0.0411 ± 0.0007 | 0.0409 ± 0.0006 |
| Example 18 | 0.0414 ± 0.0019 | 0.0419 ± 0.0012 |
| Example 19 | 0.0414 ± 0.0010 | 0.0422 ± 0.0025 |
| Example 20 | 0.0423 ± 0.0026 | 0.0449 ± 0.0014 |
| Example 21 | 0.0402 ± 0.0005 | 0.0468 ± 0.0038 |

From the results of Tables 9 to 11, it can be seen that the solutions obtained according to the present invention have excellent stability without changing the content of the active ingredient at the storage condition of 65°C. Especially, it can be seen that the increase in the amount of EDTA to 0.0005 % by weight did not cause a significant change in the content of the active ingredient (Examples 8 and 9). And also, it can be seen that, even when TED and DTPA were used instead of EDTA, there was no significant change in the content of the active ingredient (Examples 10 to 21).

### Example 22. Evaluation of nail permeability

The nail permeabilities of the solutions prepared in Examples 10 and 16 were evaluated, in a Franz diffusion cell (Labfine) for measuring nail permeability, using the finger nails from adult human cadavers. As a control formulation, JUBLIA^{™} (efinaconazole 10% containing-topical solution, Kalen Pharmaceutical Co., Ltd.) was used. The finger nails from different adult human cadavers were obtained from Science Care, 3836 E. Watkins St. Phoenix, AZ 85034 and kept in a closed container at -70°C until use. One day before the experiment, the nail plates were thawed at room temperature for 12 hours more. On the day of experiment, the nail plates were hydrated in normal saline for 3 hours at room temperature. The hydrated nails were firmly fixed between two Teflon gaskets and mounted in Franz diffusion cells (permeation area: 28.2743 mm²). The receiver compartments (i.e., acceptor compartments) were filled with 5 mL of phosphate buffered saline (pH 7.4) containing 4% (w/v) bovine serum albumin and 0.01% (w/v) sodium azide and then placed in an incubator at 32°C. And then, 50 µL of the test solutions (i.e., the solution of Example 10, the solution of Example 16, and JUBLIA^{™}, n=4 for each solution) were applied to the respective donor compartments, once a day for 7 consecutive days. On the first day, 200 µL of the samples from the respective receiver compartments were taken at 1, 2, 4, 6, 8, 20, and 24 hours from the application of the test solutions. From Day 2, the samples were taken at every 24 hours. After taking the samples, the receiver compartments were supplemented with the same volume of phosphate buffered saline (pH 7.4) containing 4% (w/v) bovine serum albumin and 0.01% (w/v) sodium azide. The obtained samples were subject to the LC/MS quantitative analyses.

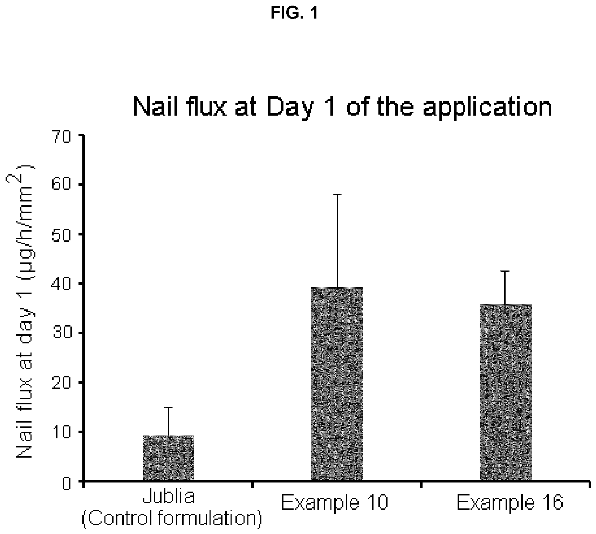

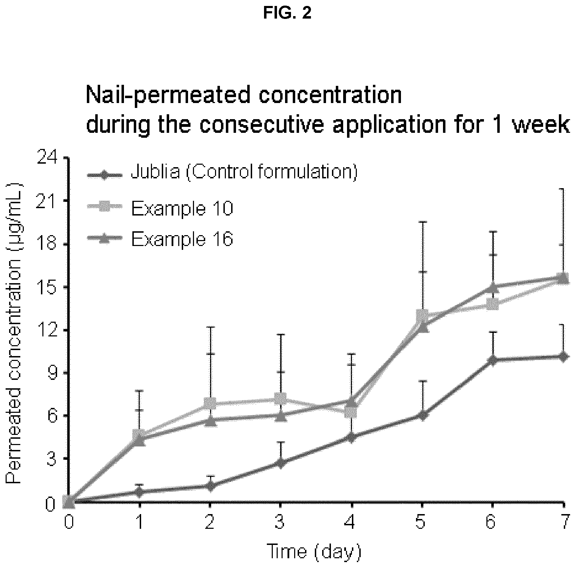

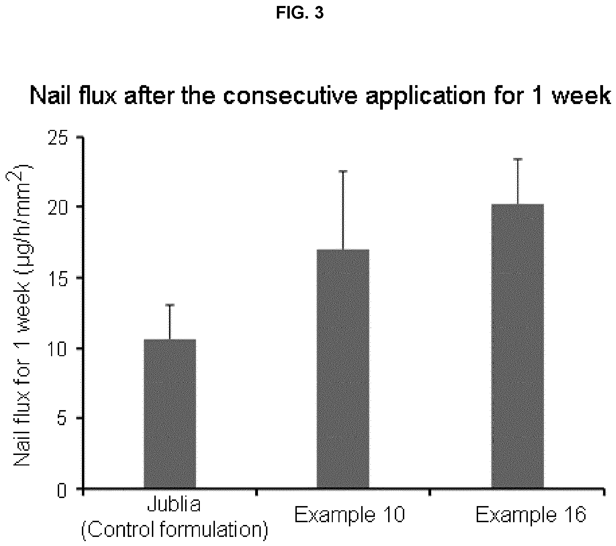

The nail fluxes calculated based on the changes in the nail-permeated concentrations at the first day of application are shown in FIG. 1. And also, the changes in the nail-permeated concentrations during the cumulative applications for 1 week are as shown in FIG. 2, and the nail fluxes calculated based on the changes are shown in FIG. 3. As can be seen from the results of FIGs. 1 to 3, in case of the solution of Example 10, efinaconazole was rapidly permeated through the nail from on the first day of application compared with the control formulation. That is, the permeated concentration and flux at 24 hours of the application thereof were increased 6.66 times and 4.24 times, respectively; and the permeated concentration and flux after one week of the consecutive application were increased to 1.53 times and 1.60 times, respectively. And also, in case of the solution of Example 16, efinaconazole was rapidly permeated through the nail from on the first day of application compared with the control formulation. That is, the permeated concentration and flux at 24 hours of the application thereof were increased 6.31 times and 3.87 times, respectively; and the permeated concentration and flux after one week of the consecutive application were increased to 1.53 times and 1.90 times, respectively. Therefore, the efinaconazole-containing formulations for external use obtained according to the present invention exhibit rapid and remarkably high nail permeability of efinaconazole, in comparison with the control formulation.

## Claims

1. A pharmaceutical composition for topical administration in the form of solution comprising efinaconazole, ethanol, cyclomethicone, and a non-volatile solvent, **characterized in that** the non-volatile solvent is diethylene glycol monoethyl ether; or a combination of diethylene glycol monoethyl ether and isopropyl myristate.

2. The pharmaceutical composition according to claim 1, wherein the non-volatile solvent is diethylene glycol monoethyl ether and the diethylene glycol monoethyl ether is present in an amount ranging from 8 to 20 % by weight, based on the total weight of the composition.

3. The pharmaceutical composition according to claim 1, wherein the non-volatile solvent is a combination of diethylene glycol monoethyl ether and isopropyl myristate, the diethylene glycol monoethyl ether is present in an amount ranging from 8 to 20 % by weight, based on the total weight of the composition, and the isopropyl myristate is present in an amount ranging from 5 to 25 % by weight, based on the total weight of the composition.

4. The pharmaceutical composition according to claim 1, wherein the non-volatile solvent is a combination of diethylene glycol monoethyl ether and isopropyl myristate, the diethylene glycol monoethyl ether is present in an amount ranging from 10 to 15 % by weight, based on the total weight of the composition, and the isopropyl myristate is present in an amount ranging from 6 to 20 % by weight, based on the total weight of the composition.

5. The pharmaceutical composition according to claim 1, comprising
8 to 12 % by weight of efinaconazole,
50 to 65 % by weight of ethanol,
8 to 20 % by weight of cyclomethicone, and
8 to 20 % by weight of diethylene glycol monoethyl ether.

6. The pharmaceutical composition according to claim 1, comprising
8 to 12 % by weight of efinaconazole,
40 to 65 % by weight of ethanol,
8 to 20 % by weight of cyclomethicone,
8 to 20 % by weight of diethylene glycol monoethyl ether, and
5 to 25 % by weight of isopropyl myristate.

7. The pharmaceutical composition according to claim 1, comprising
8 to 12 % by weight of efinaconazole,
40 to 65 % by weight of ethanol,
8 to 20 % by weight of cyclomethicone,
10 to 15 % by weight of diethylene glycol monoethyl ether, and
6 to 20 % by weight of isopropyl myristate.

8. The pharmaceutical composition according to any one of claims 1 to 7, further comprising one or more excipients selected from the group consisting of a chelating agent, an antioxidant, and an acid.

9. The pharmaceutical composition according to claim 8, wherein the chelating agent is selected from the group consisting of ethylenediaminetetraacetic acid or a sodium salt thereof, tetrahydroxypropyl ethylenediamine, and diethylenetriaminepentaacetic acid.

10. The pharmaceutical composition according to claim 8, wherein the chelating agent is tetrahydroxypropyl ethylenediamine or diethylenetriaminepentaacetic acid.

11. The pharmaceutical composition according to claim 8, wherein the antioxidant is butylated hydroxyanisole or butylated hydroxytoluene.

12. The pharmaceutical composition according to claim 8, wherein the acid is sorbic acid or citric acid.

13. The pharmaceutical composition according to claim 1, comprising
8 to 12 % by weight of efinaconazole,
50 to 65 % by weight of ethanol,
8 to 20 % by weight of cyclomethicone,
8 to 20 % by weight of diethylene glycol monoethyl ether,
0.00025 to 0.05 % by weight of a chelating agent,
0.1 to 0.2 % by weight of an antioxidant,
0.1 to 0.2 % by weight of an acid, and
0 to 1.0 % by weight of water.

14. The pharmaceutical composition according to claim 1, comprising
8 to 12 % by weight of efinaconazole,
40 to 65 % by weight of ethanol,
8 to 20 % by weight of cyclomethicone,
8 to 20 % by weight of diethylene glycol monoethyl ether,
5 to 25 % by weight of isopropyl myristate,
0.00025 to 0.05 % by weight of a chelating agent,
0.1 to 0.2 % by weight of an antioxidant,
0.1 to 0.2 % by weight of an acid, and
0 to 1.0 % by weight of water.

15. The pharmaceutical composition according to claim 1, comprising
8 to 12 % by weight of efinaconazole,
40 to 65 % by weight of ethanol,
8 to 20 % by weight of cyclomethicone,
10 to 15 % by weight of diethylene glycol monoethyl ether,
6 to 20 % by weight of isopropyl myristate,
0.00025 to 0.05 % by weight of a chelating agent,
0.1 to 0.2 % by weight of an antioxidant,
0.1 to 0.2 % by weight of an acid, and
0 to 1.0 % by weight of water.

16. The pharmaceutical composition according to claim 1, consisting of
10 % by weight of efinaconazole,
52.75 % by weight of ethanol,
15 % by weight of cyclomethicone,
12 % by weight of diethylene glycol monoethyl ether,
10 % by weight of isopropyl myristate,
0.05 % by weight of tetrahydroxypropyl ethylenediamine,
0.1 % by weight of butylated hydroxytoluene, and
0.1 % by weight of citric acid.

17. The pharmaceutical composition according to claim 1, consisting of
10 % by weight of efinaconazole,
52.29975 % by weight of ethanol,
15 % by weight of cyclomethicone,
12 % by weight of diethylene glycol monoethyl ether,
10 % by weight of isopropyl myristate,
0.00025% by weight of diethylenetriaminepentaacetic acid,
0.1 % by weight of butylated hydroxytoluene,
0.1 % by weight of citric acid, and
0.5 % by weight of water.
